# EUROPÄISCHE PATENTSCHRIFT

(11) **EP 2 470 113 B1**
(45) Veröffentlichungstag und Bekanntmachung des Hinweises auf die Patenterteilung: **28.08.2013**
(21) Anmeldenummer: 10747839.8
(22) Anmeldetag: 30.07.2010
(51) Int. Cl.: A61C 13/00, B22F 3/22

(54) **VERFAHREN ZUR HERSTELLUNG VON ZAHNTEILEN AUS DENTALMETALLPULVER**
METHOD FOR PRODUCING TOOTH PARTS FROM DENTAL METAL POWDER
PROCÉDÉ DE PRODUCTION DE PIÈCES DENTAIRES À PARTIR D'UNE POUDRE D'ALLIAGE DENTAIRE

(30) Priorität: 27.08.2009 DE 102009039102
(43) Veröffentlichungstag der Anmeldung: 04.07.2012
(73) Patentinhaber: WDT-Wolz-Dental-Technik GmbH, 55566 Bad Sobernheim (DE)
(72) Erfinder: WOLZ, Stefan, 55566 Bad Sobernheim (DE)
(74) Vertreter: Götz, Georg Alois
(86) Internationale Anmeldenummer: PCT/EP2010/061119
(87) Internationale Veröffentlichungsnummer: WO 2011/023490

(56) Entgegenhaltungen:
- WO-A1-2008/087214
- WO-A1-2008/114142

## Beschreibung

Die Erfindung betrifft ein Verfahren zur Herstellung vom Zahnteilen aus Dentalmetallpulver. Unter Zahnteile im Sinne der vorliegenden Erfindung werden insbesondere Brückengerüste und Käppchen wie auch fertige Prothesen verstanden.

Stand der Technik

Zur Herstellung von Zahnteilen aus Metall wird in der Dentaltechnik nach wie vor überwiegend das Wachsausschmelzverfahren eingesetzt, bei dem auf einem Arbeitsmodell eine Wachsform modelliert wird, deren Form dem zu gießenden Gegenstand entspricht. Mit Hilfe dieser Wachsform wird ein Hohlraum in einer Gießform gebildet, der anschließend mit Metall ausgegossen wird. Dieses Verfahren ist sehr aufwendig und erfordert viel handwerkliches Geschick.

Aus diesem Grund wurden schon einige Verfahren vorgeschlagen, die nicht mit geschmolzenem Metall sonder mit Metallpulver arbeiten, das gesintert wird. Keines dieser Verfahren hat sich aus den verschiedensten Gründen in der Praxis durchgesetzt.

Ein erfolgversprechendes Verfahren ist jedoch in der EP 1 885 278 B1 (Wolz) offenbart. Hierbei wird durch Elektrophorese aus einer Suspensionsflüssigkeit eine Metallschicht auf ein Modell niedergeschlagen, wobei die niedergeschlagene Metallschicht beim Sintern dadurch stabilisiert wird, indem sie entweder auf einem Brennträger fixiert wird, oder indem sie auf einem dublierten Modell verbleibt, oder in eine mit Einbettmasse oder temperaturbeständigem Pulver gefüllte Muffel eingelegt wird.

Neben der Herstellung von Zahnteilen aus Metall ist die Herstellung vollkeramische Zahnprothesen bekannt, die etwa 10% des Dentalmarktes abdeckt. Ein übliches Verfahren besteht darin, aus einem isostatisch vorgepreßten Block aus Keramik, insbesondere Zirkonoxid, mit der CAD/CAM-Technik ein Gerüst herauszufräsen. Hierbei wird das Gebiss des Patienten oder ein Arbeitsmodell gescannt und auf der Basis des gescannten Modells das Gerüst gefräst. Mit diesem Verfahren werden auch Zahnteile aus massiven Metallblöcken herausgefräst. Ein großer Nachteil dieses Verfahrens liegt unter anderem darin, dass das Fräsen von Keramik mit Staub verbunden ist und hohe mechanische Anforderungen an die Fräsmaschine stellt. Einfache, das heißt billige Fräsmaschinen haben daher eine zu geringe Standzeit. Selbst aufwendigere Fräsmaschinen, deren Investitionskosten oft die Wirtschaftskraft eines durchschnittlichen Zahnlabors übersteigt, erfordern einen nicht vertretbaren Wartungsaufwand. Aus diesen Gründen wurden schon in vielen Zahnlabors Fräsmaschinen stillgelegt.
WO2008/114142 A1 offenbart ein solches Verfahren, wobei Zahnteile aus Keramikpulver hergestellt werden. WO 2008/087214 A1 offenbart ein Verfahren zur Herstellung eines porösen Implantates, wobei kein Fräsen des Rohlings durchgeführt wird.

Aufgabenstellung

Es ist daher Aufgabe der in Anspruch 1 angegeben Erfindung die in Zahnlabors vorhandenen Fräsmaschinen zur Fertigung von Zahnteilen aus Metall einsetzen zu können, deren Qualität allen Anforderungen der Zahnmedizin entspricht.

Ausführungsbeispiel

Nachfolgend wird die Erfindung anhand der Herstellung von Zahnteilen aus einer CrCo-Legierung näher erläutert. Die Erfindung ist jedoch nicht auf CrCo-Legierungen beschränkt, sondern ist bei allen anderen NE-Legierungen und Edelmetall-Legierungen anwendbar.

Bei der Erfindung wird von einem Schlicker aus CrCo-Dentalmetallpulver ausgegangen. In der EP 1885 278 B1 (Wolz) ist die Herstellung eines Dentalmetallschlickers erstmals beschrieben. Ein verbesserter CrCo-Schlicker ist unter dem Namen WOLCERAM CrCo-Schlicker käuflich, der hier eingesetzt wird. Er besteht aus 98 Gew.% CrCo-Pulver und 2 Gew.% Wasser (Suspensionsflüssigkeit) mit geringen Zusätzen an organischen Verbindungen und Konservierungsmittel. Bevor dieser Schlicker verwendet wird, ist eine Konditionierung des Schlickers dringend anzuraten, um Agglomerate zu verhindern. Eine entsprechende Vorrichtung hierzu ist in der deutschen Patentschrift DE 10 2005 023 737 B4 (Wolz) offenbart. Nach der Konditionierung wird eine Form aus Silikongummi gefüllt, um eine runde Scheibe mit einem Durchmesser von 95mm und eine Dicke von 20mm herzustellen. Die Verfestigung des Schlickers zu einem brauchbaren Rohling (Grünkörper) erfolgt in der Form durch Trocknung. Um sicher zu gehen, dass keine Luftblasen in dem Schlicker vorhanden sind, wird noch eine Druckbehandlung bei 6 bar durchgeführt, durch die Luft aus dem Schlicker gepresst wird. Anschließend erfolgt der Trockungsvorgang, indem die Form in einen Trocknungsschrank bei 65°C für 7h getrocknet wird. Die Trocknung kann auch unter Vakuum und/oder unter Pressdruck durchgeführt werden. Empfehlenswert ist es, den Boden der Form mit einer wasseradsorpierenden Schicht (z.B. Löschpapier) auszulegen, damit das Wasser nicht nur an der Oberfläche, sondern auch an der Unterseite des Schlickers entweichen kann. Dadurch wird der entstehende Konzentrationsgradient im Wassergehalt reduziert, was ein Aussteigen der nichtwässrigen Hilfsstoffe an die Oberfläche verhindert. Das Ergebnis der Trocknung ist ein scheibenförmiger Grünling, der der Form der Rohlinge entspricht, die in übliche CAD/CAM-Fräsmaschinen bearbeitet werden. Aus dem massiven Rohling (Grünling) werden ca. 30 Zahnteile von beiden Seiten herausgefräst, die noch durch Stege verbunden sind. Nach dem Entfernen der Stege liegen 30 Zahnteile vor. Im Gegensatz zum Fräsen aus einer Metallscheibe ist beim vorliegenden Verfahren das Entfernen der Stege und das Glätten der Stegstellen leicht durchzuführen, da das Material noch ziemlich weich ist.

Die erhaltenen grünen Zahnteile werden anschließend einer sauerstofffreien Sinterung unterzogen. Hierzu werden sie in einen Sinterofen eingebracht, wie er in der älteren Patentanmeldung 10 2009 037 737.9 (Anmeldetag 17.08.2009) offenbart ist. Die Sinterung der grünen CrCo-Zahnteile erfolgt bei 1190°C und eine Dauer von 45 min. Zum Verdrängen des Sauerstoffes werden 2,5 l/min Argon eingeleitet. Das Ergebnis der Sinterung sind metallisch glänzende Zahnteile aus CrCo-Metall, die, wie üblich, noch verblendet werden können. Es versteht sich von selbst, dass der beim Sintern entstehende Schrumpf durch ein entsprechendes CAM/CAD-Programm kompensiert werden muss.

Das gesinterte Produkt weist folgende Eigenschaften aus. Die chemische Zusammensetzung wurde emissionsspektroskopisch ermittelt. Co-%

| | | | | |
|---|---|---|---|---|
| Rest | Mn-% | <1,0 Cr-% | 28,0-30,0 | Si-% |
| <1,0 Mo-% | 5,0-6,0 | Fe - C - Ni | Spuren | <0,5% |

| | |
|---|---|
| Technische Daten: Dichte (g/cm³) | 8,3 |
| mittlerer linearer WAK 25-500 °C (10⁻⁶ K⁻¹) | 14,5 |
| E-Modul (GPa) | 228,7 0,2% Dehngrenze |
| (MPa) | 817 Bruchdehnung |
| (%) | 9,7 Vickershärte (HV5/30) |
| | 375 Chemische Löslichkeit |
| (µg/cm²) | < 4 |

Normen: ISO 9693:1999; ISO 22674:2006; ISO 10993-5:1999

Da einige Fräsmaschinen mit Wasser als Kühlmittel arbeiten, kann sich ein Problem dadurch ergeben, dass das Rohmaterial noch wasserlöslich ist. Nach einer Temperung des Rohlings bei 250-400 °C für 2 bis 3 Stunden kann das Material bei Anwesenheit von Wasser ohne Schwierigkeiten gefräst werden. Das Material ist in wässriger Umgebung dann stabil.

## Patentansprüche

1. Verfahren zur Herstellung von Zahnteilen aus Dentalmetallpulver mit den folgenden Verfahrensschritten: a) Bereitstellung eines Schlickers aus Dentalmetallpulver, b) Eingießen dieses Schlickers in eine Form, c) Entziehung von Suspensionsflüssigkeit aus dem eingegossenen Schlicker, bis ein mechanisch stabiler Rohling entsteht, d) Fräsen des Rohlings in die gewünschte Raumform, e) sauerstofffreies Sintern der aus dem Rohling herausgefrästen Zahnteile.

2. Verfahren nach Anspruch 1, wobei die Suspensionsflüssigkeit Wasser ist, das durch Trocknung entzogen wird.

3. Verfahren nach Anspruch 1 oder 2, wobei die Entziehung der Suspensionflüssigkeit auch am Boden der Form durch eine adsorbierende Schicht erfolgt.

4. Verfahren nach einem der Ansprüche 1 bis 3, wobei das Dentalmetallpulver aus einer CrCo-Legierung besteht.

5. Verfahren nach einem der Ansprüche 1 bis 4, wobei der Rohling bei 250 bis 500 °C getempert wird.

## Claims

1. Method for producing tooth parts from dental-grade metal powder with the following process steps:
a) preparing a slip of dental-grade metal powder,
b) casting this slip into a mold,
c) drawing suspension liquid out of the cast slip until a mechanically stable blank is obtained,
d) milling the blank to the desired shape,
e) oxygen-free sintering the tooth parts that have been milled from the blank.

2. Method according to claim 1, wherein the suspension liquid is water, which is drawn out by drying.

3. Method according to claim 1 or 2, wherein the suspension liquid is also drawn out at the base of the mold by way of an absorbent layer.

4. Method according to any one of claims 1 to 3, wherein the dental-grade metal powder consists of a CrCo alloy.

5. Method according to any one of claims 1 to 4 wherein the blank is tempered at 250 to 500 °C.

## Revendications

1. Procédé de fabrication de pièces dentaires à partir d'une poudre métallique pour applications dentaires, comportant les étapes suivantes : a) mise à disposition d'une barbotine de poudre métallique pour applications dentaires, b) introduction par coulée de ladite barbotine dans un moule, c) extraction du liquide de suspension de la barbotine introduite par coulée, jusqu'à obtention d'une ébauche mécaniquement stable, d) fraisage de l'ébauche pour obtenir la forme tridimensionnelle souhaitée, e) frittage sans oxygène des pièces dentaires obtenues par fraisage à partir de l'ébauche.

2. Procédé selon la revendication 1, **caractérisé en ce que** le liquide de suspension est de l'eau qui a été extraite par séchage.

3. Procédé selon la revendication 1 ou 2, dans lequel l'extraction du liquide de suspension a lieu aussi au fond du moule grâce à une couche adsorbante.

4. Procédé selon l'une des revendications 1 à 3, dans lequel la poudre métallique pour applications dentaires est constituée d'un alliage CrCo.

5. Procédé selon l'une des revendications 1 à 4, dans lequel l'ébauche est recuite entre 250 et 500°C.
